# EUROPEAN PATENT APPLICATION

(11) **EP 4 321 163 A1**
(43) Date of publication of application: **14.02.2024**
(21) Application number: 22784600.3
(22) Date of filing: 30.03.2022
(51) Int. Cl.: A61K 31/4965, A61P 31/14

(54) **THERAPEUTIC AGENT FOR CORONAVIRUS INFECTION**

(30) Priority: 08.04.2021 JP 2021065740
(71) Applicant: FUJIFILM Toyama Chemical Co., Ltd., Chuo-ku Tokyo 104-0031 (JP)
(72) Inventor: SAKURAI Tsutomu, Tokyo 104-0032 (JP); YAMATAKE Takahiro, Tokyo 104-0032 (JP)
(74) Representative: Wächtershäuser & Hartz Patentanwaltspartnerschaft mbB
(86) International application number: PCT/JP2022/015829
(87) International publication number: WO 2022/215616

(57) **Abstract**

The present invention makes it possible to treat a coronavirus infection by administering a pharmaceutical composition containing 6-fluoro-3-hydroxy-2-pyrazinecarboxamide or a salt thereof as an active ingredient to a patient infected with the coronavirus who has an aggravating risk factor and is in an early stage of the disease.

## Description

### TECHNICAL FIELD

The present invention relates to a therapeutic agent for coronavirus infection comprising 6-fluoro-3-hydroxy-2-pyrazinecarboxamide (nonproprietary name, favipiravir; hereinafter referred to as Compound A) or a salt thereof as an active ingredient.

### BACKGROUND ART

The novel coronavirus (SARS-CoV-2) first reported in China at the end of 2019 (Non Patent Document 1) is an RNA virus, which belongs to the family coronaviridae, order nidovirales (Non Patent Document 2). When infected with this virus, fever and acute respiratory symptoms including cough and dyspnea develop (Non Patent Document 1), and when the condition is exacerbated, pneumonia develops. As of March 17, 2021, there had been more than 440,000 people testing positive for SARS-CoV-2 in Japan and more than 8,600 deaths among them (Non Patent Document 3).

Compound A is an antiviral drug developed by FUJIFILM Toyama Chemical Co., Ltd. (previously Toyama Chemical Co., Ltd.) and was approved for manufacturing and marketing in March 2014 in Japan for an indication limited to "novel or re-emerging influenza virus infection (only for cases in which other anti-influenza virus agents were not effective or were insufficiently effective)." Since the mechanism thereof is that the triphosphate oxide form (T-705 RTP) converted in vivo selectively inhibits the viral RNA polymerase, Compound A may be effective on other RNA viruses than influenza virus. In fact, Compound A has been reported to have effects against Ebola virus and RNA viruses belonging to the family Arenaviridae and the family Bunyaviridae in vitro or in vivo (Non Patent Documents 4, 5, 6).

Additionally, a Japanese clinical phase III study of Compound A in COVID-19 patients with non-serious pneumonia has demonstrated with a statistically significant difference that time from the start of administration of the study drug to a time point where body temperature, percutaneous arterial blood oxygen saturation (SpO₂), and chest image findings are improved and SARS-CoV-2 is tested negative conversion, the primary endpoint, was shorter by approximately 3 days in the Compound A group than in the control group, indicating that administration of Compound A to COVID-19 patients with non-serious pneumonia improves their symptoms early (Non Patent Document 7).

### PRIOR ART DOCUMENT

### NON PATENT DOCUMENT

**Non Patent Document 1:** Wang C, Horby PW, Hayden FG, Gao GF. A novel coronavirus outbreak of global health concern. Lancet. 2020;395:470-3.
**Non Patent Document 2:** Coronaviridae Study Group of the International Committee on Taxonomy of Viruses. The species Severe acute respiratory syndrome-related coronavirus: classifying 2019-nCoV and naming it SARS-CoV-2. Nat Microbiol. 2020 Mar 2. PubMed PMID: 32123347.
**Non Patent Document 3:** Kokunai no hassei jokyo nado (in Japanese) (Situation of occurrences etc. in Japan). (Updated 17 Mar 2021; cited 18 Mar 2021). [Internet]. Tokyo: Ministry of Health, Labour and Welfare. Available from: https://www.mhlw.go.jp/stf/covid-19/kokunainohasseijoukyou.html
**Non Patent Document 4:** Gowen BB, Wong MH, Jung KH, Sanders AB, Mendenhall M, Bailey KW, et al. In vitro and in vivo activities of T-705 against arenavirus and bunyavirus infections. Antimicrob Agents Chemother. 2007;51:3168-76.
**Non Patent Document 5:** Mendenhall M, Russell A, Smee DF, Hall JO, Skirpstunas R, Furuta Y, et al. Effective oral favipiravir (T-705) therapy initiated after the onset of clinical disease in a model of arenavirus hemorrhagic Fever. PLoS Negl Trop Dis. 2011;5:e1342.
**Non Patent Document 6:** Oestereich L, Ludtke A, Wurr S, Rieger T, Munoz-Fontela C, Gunther S. Successful treatment of advanced Ebola virus infection with T-705 (favipiravir) in a small animal model. Antiviral Res. 2014;105:17-21.
**Non Patent Document 7:** "The primary endpoint was achieved in a Japanese clinical phase III study in patients with novel coronavirus infection" (News release dated 23 September 2020). [Internet]. Fujifilm Co., Ltd. Available from: https://www.fujifilm.com/jp/ja/news/list/5451

### SUMMARY OF INVENTION

### TECHNICAL PROBLEM

No treatment methods have been established for coronavirus infection, and there is a need to develop a therapeutic agent for coronavirus infection early. In Japan, remdesivir, an antiviral drug, was approved for an indication of COVID-19 and is used in patients with pneumonia caused by SARS-CoV-2. However, there are no available antiviral drugs that can be used in patients with a risk factor for disease progression at an early stage of infection. An object of the present invention is to provide a therapeutic agent for coronavirus infection that can delay the progression of symptoms by using it in patients with a risk factor for disease progression at an early stage after onset, and a method for treating coronavirus infection.

### SOLUTION TO PROBLEM

Under the above-described circumstances, the present inventors have found that coronavirus infection can be treated by administering Compound A or a salt thereof to coronavirus-infected patients with a risk factor for disease progression at an early stage after onset, and accomplished the present invention.

According to the present invention, the following inventions are provided.
[1] A therapeutic agent for coronavirus infection comprising 6-fluoro-3-hydroxy-2-pyrazinecarboxamide or a salt thereof as an active ingredient, which is administered to patients with one or more risk factors for disease progression who are at an early stage after onset.
[2] The therapeutic agent for coronavirus infection according to [1], wherein the patients are patients with novel coronavirus infection.
[3] The therapeutic agent for coronavirus infection according to [1] or [2], wherein the risk factor for disease progression is selected from the group consisting of advanced age (65 years of age or older), malignant tumor, COPD, heart disease, chronic lung disease, cerebrovascular disorder, chronic kidney disease diagnosed and under treatment, type 2 diabetes mellitus, hypertension or hyperlipidemia, obesity (BMI 30 kg/m² or higher), and smoking habit.
[4] The therapeutic agent for coronavirus infection according to any of [1] to [3], wherein the onset means that at least one selected from the group consisting of cough, sore throat, headache, nasal obstruction or rhinorrhea, myalgia or arthralgia, fatigue or tiredness, dysgeusia, anosmia, and diarrhea occurs as the first symptom.
[5] The therapeutic agent for coronavirus infection according to any of [1] to [4], wherein the early stage after onset means within 72 hours from onset of the first symptom.
[6] The therapeutic agent for coronavirus infection according to any of [1] to [5], wherein the patients are 50 years of age or older.
[7] The therapeutic agent for coronavirus infection according to any of [1] to [6], wherein 6-fluoro-3-hydroxy-2-pyrazinecarboxamide is administered at 1,000 to 2,400 mg twice a day on Day 1 and at 400 to 1,200 mg twice a day on Day 2 and thereafter.
[8] The therapeutic agent for coronavirus infection according to any of [1] to [7], wherein 6-fluoro-3-hydroxy-2-pyrazinecarboxamide is administered at 1,800 mg twice a day on Day 1 and at 800 mg twice a day on Day 2 and thereafter.
[9] The therapeutic agent for coronavirus infection according to any of [1] to [8], which is administered for 10 days.

According to the present invention, the following inventions are also provided:
(a) A pharmaceutical composition comprising Compound A or a salt thereof for the treatment of coronavirus infection, the pharmaceutical composition administered to patients with one or more risk factors for disease progression who are at an early stage after onset.
(b) Compound A or a salt thereof for the treatment of coronavirus infection, the Compound A or a salt thereof administered to patients with one or more risk factors for disease progression who are at an early stage after onset.
(c) A method for treating coronavirus infection comprising administration of Compound A or a salt thereof, the method comprising administration to patients with one or more risk factors for disease progression who are at an early stage after onset.
(d) Use of Compound A or a salt thereof to manufacture a therapeutic agent for coronavirus infection administered to patients with one or more risk factors for disease progression who are at an early stage after onset.

### ADVANTAGEOUS EFFECTS OF INVENTION

Coronavirus infection can be treated by administering Compound A or a salt thereof to coronavirus-infected patients with one or more risk factors for disease progression who are at an early stage after onset.

### DESCRIPTION OF EMBODIMENTS

The present invention will be described in detail below.

In this specification, each term has the following meaning unless otherwise specified.

In this specification, a range of numerical values represented using "to" means a range including the numerical values before and after "to" as the minimum value and the maximum value, respectively. In an aspect, the value of either one or both of the minimum value and the maximum value may be excluded (in other words, "x or more/higher" can mean "more than x" and "x or less/lower" can mean "less/lower than x").

In the present specification, when a plurality of substances corresponding to each component exist in the composition, the amount of each component in the composition means the total amount of the plurality of substances in the composition, unless otherwise specified.

Compound A means 6-fluoro-3-hydroxy-2-pyrazinecarboxamide.

Examples of a salt of Compound A include commonly known salts in basic groups such as an amino group and acidic groups such as a hydroxyl group or a carboxyl group.

Examples of the salts in basic groups include salts with mineral acids such as hydrochloric acid, hydrobromic acid, nitric acid, and sulfuric acid; salts with organic carboxylic acids such as formic acid, acetic acid, citric acid, oxalic acid, fumaric acid, maleic acid, succinic acid, malic acid, tartaric acid, aspartic acid, trichloroacetic acid and trifluoroacetic acid; and salts with sulfonic acids such as methanesulfonic acid, benzenesulfonic acid, p-toluenesulfonic acid, mesitylenesulfonic acid, and naphthalenesulfonic acid.

Examples of the salts in acidic groups include salts with alkali metals such as sodium and potassium; salts with alkaline earth metals such as calcium and magnesium; ammonium salts; and salts with nitrogen-containing organic basic compounds such as trimethylamine, triethylamine, tributylamine, pyridine, *N*,*N*-dimethylaniline, *N*-methylpiperidine, N-methylmorpholine, diethylamine, dicyclohexylamine, procaine, dibenzylamine, *N*-benzyl-β-phenethylamine, 1-efenamine, *N*,*N*'-dibenzylethylenediamine, and meglumine.

Among the above-mentioned salts, examples of preferred salts include pharmacologically acceptable salts, and examples of more preferred salts include salts with sodium or meglumine.

If Compound A or a salt thereof has isomers (e.g., optical isomers, geometrical isomers, and tautomers), the present invention encompasses all these isomers, as well as hydrates, solvates, and all crystal forms.

The term "coronavirus" used in this specification means an RNA virus belonging to the family coronaviridae, order nidovirales. Coronavirus infection is an infectious disease caused by coronavirus. The term "novel coronavirus" means a newly identified and reported novel coronavirus among coronaviruses, and examples thereof include SARS-CoV-2 reported at the end of 2019. The infectious disease caused by SARS-CoV-2 is also called COVID-19. The term "novel coronavirus" naturally encompasses mutants and variants of the known coronavirus. Examples of variants of SARS-CoV-2 include VOC-202012/01, 501Y.V2, and 501YV3.

Treatment means relieving or improving one or more symptoms caused by a specific disease that affects a target patient, as well as delaying the progression of the disease. In an embodiment of the present invention, for example, treatment means delaying disease progression in patients with coronavirus infection or relieving or improving symptoms, such as pyrexia, cough, and pneumonia. In an embodiment of the present invention, treatment means improving body temperature, percutaneous arterial blood oxygen saturation (SpO₂), and chest image findings or achieving negative conversion. The term "disease progression" used herein means that a patient with coronavirus infection falls into a condition requiring administration of oxygen in some way or dies.

Patients to whom Compound A or a salt thereof is administered are preferably patients with one or more risk factors for disease progression who are at an early stage after onset. Also, patients treated with Compound A or a salt thereof are preferably not patients with non-serious pneumonia. The term "non-serious pneumonia" used herein means pneumonia that does not accompany hypoxemia requiring oxygen therapy.

Examples of the risk factor for disease progression include advanced age (65 years of age or older), malignant tumor, chronic obstructive pulmonary disease (COPD), heart disease, chronic lung disease, cerebrovascular disorder, chronic kidney disease diagnosed and under treatment, type 2 diabetes mellitus, hypertension or hyperlipidemia, obesity (BMI 30 kg/m² or higher), and smoking habit.

Examples of the early stage after onset include cases where onset of the first symptom occurs within 24 hours, within 48 hours, within 72 hours, and within 96 hours, and onset within 72 hours is preferred. Of note, if specific time of onset of the first symptom is not clear, early morning may be considered as 6 o'clock, morning as 8 o'clock, lunch time as 12 o'clock, daytime as 15 o'clock, evening as 18 o'clock, before bedtime as 22 o'clock, and midnight as 0 o'clock. Examples of symptoms include cough, sore throat, headache, nasal obstruction or rhinorrhea, myalgia or arthralgia, fatigue or tiredness, dysgeusia, anosmia, and diarrhea.

Compound A or a salt thereof used in the present invention can be manufactured by using methods known per se or by using them in combination appropriately. For example, Compound A or a salt thereof can be manufactured by the methods described in International Publication No. WO 00/10569. Compound A has a tautomer 6-fluoro-3-oxo-3,4-dihydro-2-pyrazinecarboxamide.

Compound A or a salt thereof used in the present invention can be formulated as pharmaceutical preparations such as oral agents (e.g., tablets, capsules, powders, granules, subtilized granules, pills, suspensions, emulsions, liquids, syrups), injections, eye drops, nasal agents, and percutaneous agents by adding various pharmaceutical additives, such as excipients, binders, disintegrants, disintegration inhibitors, anti-caking/anti-adhesive agents, lubricants, absorption/adsorption carriers, solvents, expanders, tonicity adjusting agents, dissolving aids, emulsifiers, suspending agents, thickeners, coating agents, absorption enhancers, gelling/solidifying enhancers, light stabilizers, preservatives, desiccating agents, emulsion/suspension/dispersion stabilizers, discoloration-preventive agents, oxygen absorbers/antioxidants, taste masking/odor masking agents, coloring agents, foaming agents, defoaming agents, soothing agents, antistatic agents, and buffers/pH adjusters. Preparations administered to patients with novel coronavirus infection are preferably oral agents or injections, more preferably oral agents, yet more preferably tablets.

The above-mentioned pharmaceutical agents are formulated by usual methods.

The method of administering Compound A is not particularly limited and is suitably determined depending on the form of the preparations, the patient's age, sex, and other conditions, and the severity of the patient's symptoms.

The above-described Japanese clinical phase III study of Compound A in COVID-19 patients with non-serious pneumonia has shown that symptoms can be improved early by administering Compound A at 1,800 mg twice a day on Day 1 and at 800 mg twice a day on Day 2 and thereafter for maximum 14 days, in addition to the standard of care for viral pneumonia.

The dose of Compound A is suitably selected depending on the dosing regimen, the patient's age and sex, disease form, and other conditions. For example, Compound A can be administered to adults at 10 to 5,000 mg or preferably 200 to 2,400 mg once or divided into several doses a day. Compound A can be administered as multiple doses or a single dose until the desired therapeutic effect is achieved. The administration is monitored preferably by inpatient management, and Compound A can be administered repeatedly, if necessary.

In the present invention, Compound A can be administered to adults at 1,000 to 2,400 mg twice a day on Day 1 and at 400 to 1,200 mg twice a day on Day 2 and thereafter. Compound A can be administered to adults preferably at 1,600 mg twice a day on Day 1 and at 600 mg twice a day on Day 2 and thereafter and Compound A can be administered to adults at 1,800 mg twice a day on Day 1 and at 800 mg twice a day on Day 2 and thereafter, and Compound A can be administered more preferably at 1,800 mg twice a day on Day 1 and at 800 mg twice a day on Day 2 and thereafter.

The interval between the first and second doses for the twice a day administration is preferably an interval of at least 4 hours on Day 1 and an interval of 12 hours on Day 2 and thereafter.

The administration period is suitably determined depending on changes overtime in symptoms, and for example, can be selected from up to 5 days, 6 days, 7 days, 8 days, 9 days, 10 days, 11 days, 12 days, 13 days, 14 days, 15 days, 16 days, 17 days, 18 days, 19 days, 20 days, 21 days, and 22 days. The administration period is preferably up to 8 days, 9 days, 10 days, 11 days, 12 days, 13 days, or 14 days, and up to 10 days is more preferred.

In the present invention, the administration of Compound A or a salt thereof can include administration of a concomitant drug and/or performing a concomitant therapy. Examples of the concomitant drug and/or concomitant therapy include the following:
(1) Therapeutic agents for COVID-19 (e.g., remdesivir, dexamethasone)
(2) Drugs reported with an antiviral effect against SARS-CoV-2 (e.g., hydroxychloroquine sulfate, chloroquine phosphate, lopinavir/ritonavir combination, ciclesonide, nafamostat mesylate, camostat mesylate, nelfinavir mesylate, ivermectin, interferons, interferon-beta, fluvoxamine, colchicine, vitamin D, zinc, famotidine, sofosbuvir, daclatasvir)
(3) Preparations containing an adrenocorticosteroid
(4) Tocilizumab, sarilumab, baricitinib, adrenomedullin, sargramostim
(5) Vaccines against SARS-CoV-2
(6) Hemocatharsis therapy (e.g., hemodialysis, blood filtration, hemodialysis filtration, blood adsorption, plasma exchange, plasma adsorption, peritoneal dialysis)

The present invention will be described using a clinical trial as an example, but the present invention is not limited to the example.

Test example 1: A placebo-controlled, stratified-randomized, multicenter, double-blind study to evaluate the efficacy and safety of Compound A in COVID-19 patients with a risk factor for disease progression who are at an early stage after onset

### [Methodology] 1. Study design

### 1.1 Type of study

Confirmatory study (phase 3)

### 1.2 Study design

Placebo-controlled, stratified-randomized, multicenter, double-blind design

### 1.3 Group composition and administration methods

### [Compound A group]

Compound A 1,800 mg twice/day for 1 day + 800 mg twice/day for 9 days

### [Placebo group]

Compound A placebo 9 tablets twice/day for 1 day + 4 tablets twice/day for 9 days

### 1.4 Endpoints

### 1.4.1 Primary efficacy endpoint

Proportion of patients who experienced disease progression during the period between randomization and Day 28

Disease progression is defined as a case where the patient's condition (score) has become 4 to 7 based on the 7-point scale defined in Table 1.

**[Table 1]**

| Score | Definition |
|---|---|
| 1 | No symptom |
| 2 | Condition with a symptom (fever of 37.5 ° C or higher, cough, sore throat, nasal obstruction or rhinorrhea, myalgia or arthralgia, fatigue or tiredness, headache, diarrhea, dysgeusia, or anosmia) |
| 3 | Condition with dyspnea or shortness of breath and requiring a rest, but not requiring supplemental oxygen therapy |
| 4 | Condition requiring supplemental oxygen therapy (not applicable to oxygen therapy for sleep apnea syndrome) |
| 5 | Condition requiring noninvasive mechanical ventilation therapy or high flow oxygen therapy |
| 6 | Condition requiring invasive mechanical ventilation therapy or ECMO |
| 7 | Death |

| | |
|---|---|
| Modified Ordinal Scale for Clinical Improvement from WHO R&D Blueprint Novel Coronavirus COVID-19 Therapeutic Trial Synopsis ECMO: extracorporeal membrane oxygenation | |

### 1.4.2 Secondary efficacy endpoints

(1) Proportion of patients who experienced disease progression before Day 15
(2) Change overtime in SARS-CoV-2 genome amount
(3) Time to recovery to negative conversion of SARS-CoV-2
(4) Change overtime in the proportion of patients testing negative for SARS-CoV-2
(5) Time to disease progression
(6) Change overtime in the patient's condition based on the 7-point scale
(7) Change overtime in vital signs (SpO₂, body temperature, respiratory rate)
(8) Change overtime in the proportion of patients with worsened clinical symptoms/findings
(9) Duration of oxygen administration

### 1.4.3 Pharmacokinetic and PK-PD endpoints

(1) Change overtime in plasma concentrations of Compound A
(2) Pharmacokinetic parameters of Compound A (AUC₀₋₂₄, Cₘₐₓ, Cₘᵢₙ)
(3) Relationship between pharmacokinetic parameters of Compound A and viral proliferation inhibiting effect
(4) Relationship between pharmacokinetic parameters of Compound A and adverse events

### 1.4.4 Safety endpoints

(1) Adverse events
(2) Laboratory test values
(3) Vital signs

### 1.5 Target number of patients

316 patients enrolled (158 patients in the Compound A group and 158 patients in the placebo group)

### 2. Subject

### 2.1 Subject

COVID-19 patients aged 50 years or older with the following risk factor(s) for disease progression who experienced onset of symptoms within 72 hours before the start of administration of the study drug:

### <Risk factors for disease progression>

Advanced age (65 years of age or older), malignant tumor, COPD, heart disease, chronic lung disease, cerebrovascular disorder, chronic kidney disease diagnosed and under treatment, type 2 diabetes mellitus, hypertension or hyperlipidemia, obesity (body mass index [BMI] 30 kg/m² or higher), smoking habit

### 2.2 Inclusion criteria

(1) Age: 50 years of age or older (at the time of consent)
(2) Sex: Irrespective
(3) Outpatient/hospitalization: Hospitalization
(4) Patients positive for SARS-CoV-2 based on a nucleic acid detection test (RT-PCR or loop-mediated isothermal amplification [LAMP] method) or an antigen test using specimens such as nasopharyngeal swab, nasal aspirate, a respiratory tract aspirate, or saliva
(5) Patients 50 to 64 years of age who meet all the following criteria 1) to 3) at the time of informed consent or patients 65 years of age or older who meet both the criteria 1) and 2) at the time of informed consent:
   1) SpO₂ of 96% or higher in a condition without oxygen therapy
   2) One or more of the following symptoms, and onset of the first symptom occurred within 72 hours before the start of administration of the study drug (if specific time of onset of the first symptom is not clear, early morning is considered as 6 o'clock, morning as 8 o'clock in the morning, lunch time as 12 o'clock, daytime as 15 o'clock, evening as 18 o'clock, before bedtime as 22 o'clock, and midnight as 0 o'clock):
      Pyrexia (37.5°C or higher), cough, sore throat, headache, nasal obstruction or rhinorrhea, myalgia or arthralgia, fatigue or tiredness, dysgeusia, anosmia, diarrhea
   3) One or more of the following risk factors for disease progression:
      Malignant tumor, COPD, heart disease, chronic lung disease, cerebrovascular disorder, chronic kidney disease diagnosed and under treatment, type 2 diabetes mellitus, hypertension or hyperlipidemia, obesity (BMI 30 kg/m² or higher), smoking habit
(6) When premenopausal female is targeted, patients testing negative for pregnancy before the start of administration of the study drug
(7) Patients who understand the content of this clinical trial and are able to submit written consent by himself/herself

### 2.3 Exclusion criteria

(1) Patients on oxygen therapy before the start of administration of the study drug
(2) Patients with at least one of dyspnea and a respiratory rate of 30 bpm or more as a clinical symptom/finding of COVID-19
(3) Patients who have received vaccination against SARS-CoV-2
(4) Patients who have used any of the following drugs during the period between 5 days before onset and before the start of administration of the study drug:
   1) Therapeutic agents for COVID-19 (remdesivir, dexamethasone)
   2) Drugs whose anti-viral action against SARS-CoV-2 has been reported (hydrodxychloroquine sulfate, chloroquine phosphate, lopinavir/ritonavir combination drugs, ciclesonide, nafamostat mesilate, camostat mesilate, nelfinavir mesylate, ivermectin, interferons, interferon-beta, fluvoxamine, colchicine, vitamin D, zinc, famotidine, sofosbuvir, daclatasvir)
   3) Preparations containing an adrenocorticosteroid
      Except when patients have continued to use the preparation at the same dose from 2 weeks before the start of administration of this clinical study. Use of topical agents except suppositories and inhalants is allowed.
   4) Tocilizumab, sarilumab, baricitinib, adrenomedullin, sargramostim
(5) Patients who has experienced this infection episode as a recurrence of SARS-CoV-2 infection or has a history of SARS-CoV-2 infection
(6) Patients suspected of concurrent bacterial infection based on an elevated procalcitonin value before the start of administration of the study drug or the like
(7) Patients suspected of concurrent fungal infection based on an abnormal (1→3)-β-D-glucan value before the start of administration of the study drug or the like
(8) Patients suspected of concurrent congestive heart failure based on a human N-terminal fragment of brain natriuretic peptide precursor (NT-proBNP) value of 400 pg/mL or higher (or human brain natriuretic peptide [BNP] value of 100 pg/mL or higher) before the start of administration of the study drug or the like
(9) Patients with severe hepatic impairment of Grade C according to the Child-Pugh classification
(10) Patients with renal impairment requiring dialysis
(11) Patient with consciousness disturbed including orientation disturbed
(12) Patients who are pregnant, of child-bearing potential, or wish to get pregnant
(13) Patients who are breast-feeding
(14) Pre-menopausal female patients who do not agree to exercise contraception by methods using mechanical contraceptives, such as intrauterine device or barrier method (pessary, condom), or combinations thereof and the like from the start of administration of the study drug to 14 days after the end of administration
(15) Male patients who do not agree to preventing contraception by methods using a condom, using other barrier methods (e.g., pessary) in combination and the like whenever they have sexual intercourse and not having sexual intercourse with a pregnant woman during from the start of administration of the study drug to 7 days after the end of administration
(16) Patients with hereditary xanthinuria
(17) Patients who have been diagnosed with hypouricemia (less than 1 mg/dL) or xanthine urinary calculus
(18) Patients with a history of gout or on treatment for gout or hyperuricemia
(19) Patients who have received an immunosuppressive agent or an anticancer drug with a potent myelosuppressive effect
(20) Patients who received another study drug within 90 days before the start of administration of the study drug
(21) Patients who were administered Compound A
(22) Patients who are deemed to be ineligible for the study by the principal investigator or a subinvestigator

### 3. Study drug

Tablet A: One tablet contains 200 mg of Compound A (administered to the Compound A group)
Tablet B: A tablet which is indistinguishable in appearance from Tablet A and does not contain Compound A (administered to the placebo group)

### Dosage form: Pale yellow, film-coated tablet

Tablet A and Tablet B used in this study are manufactured by using the methods described in International Publication No. WO 2010/104170 or methods known per se or using these methods in combination appropriately.

### 4. Dosing regimen, dose and administration period

### 4.1 Dosing regimen and dose

### (1) Dosage

The study drug at the following doses will be administered according to the administration group:

### [Compound A group]

Compound A 1,800 mg twice/day for 1 day + 800 mg twice/day for 9 days

### [Placebo group]

Compound A placebo 9 tablets twice/day for 1 day + 4 tablets twice/day for 9 days

### (2) Administration method

### [Compound A group]

Tablet A (containing 200 mg of Compound A) will be orally administered at 9 tablets twice/day on Day 1 and 4 tablets twice/day on Days 2 to 10.

### [Placebo group]

Tablet B (placebo not containing Compound A) will be orally administered at 9 tablets twice/day on Day 1 and 4 tablets twice/day on Days 2 to 10.

On Day 1, the second dose will be administered with at least 4 hour interval from administration of the first dose. It is administered with a 12 hour interval on Days 2 to 10, whenever possible.

### 4.2 administration period

The administration period is for 10 days.

### 5. Criteria for evaluation

### 5.1 Study procedures

The clinical trial will be conducted in accordance with the following procedures:
(1) Confirmation of inclusion/exclusion criteria, explanation of the clinical trial and obtaining informed consent, performing of observations and tests before the start of administration
(2) Registration of the patient (enrollment to the study)
(3) Notification of drug numbers (assignment to treatment)
(4) Prescription of the study drug and start of administration
(5) Specified observations and tests, evaluations/determination
(6) Follow-up investigation

### 5.2 Evaluation criteria

### 5.2.1 Efficacy evaluation criteria

### (1) Determination criteria for the primary endpoint

### 1) Determination of disease progression

When the patient's condition (score) is assessed as a score of 4 to 7 using the 7-point scale defined above in Table 1 during the period between randomization and Day 28, patients are considered as having "disease progression."

### (2) Determination criteria for secondary endpoints

### 1) Determination of negative SARS-CoV-2

"Achieving negative conversion" is determined at a time point when negativity is confirmed for the first time after the last collection of a specimen testing positive for SARS-CoV-2 by RT-PCR (qualitative) testing. If a patient is negative on all dates for specimen collection including predose, it is considered that "achieving negative conversion" occurred predose.

### 2) Determination of worsening of clinical symptom/finding

"Worsening" is determined for each clinical symptom/finding at a time point of the first observation meeting the criteria of worsening shown in Table 2. If the criteria of worsening are met at a predose time point, it is considered that "worsening" occurred predose.

**[Table 2]**

| Clinical symptom or finding | Criteria of worsening |
|---|---|
| Cough | 2+ (moderate) or 3+ (severe) |
| Sore Throat | |
| Headache | |
| Myalgia or Arthralgia | |
| Nasal Obstruction or Rhinorrhea | |
| Fatigue or Tiredness | |
| Diarrhoea | |
| Ageusia | + (Yes) |
| Anosmia | |
| Dyspnea | |
| Disorientation | V [Responsive to voice], |
| | P [Responsive to pain] or |
| | U [Unresponsive] |

### 5.2.2 Safety evaluation criteria

The principal investigator or a subinvestigator will determine the severity of adverse events that have developed during the clinical trial and the causal relationships of these adverse events. Symptoms of adverse events are evaluated by severity and grade.

### [Results]

It can be demonstrated with the primary endpoint and/or secondary endpoints that Compound A has therapeutic effect in COVID-19 patients with a risk factor for disease progression who are at an early stage after onset. Specifically, for example, when the study results are analyzed for an appropriately defined efficacy analysis target population, it can be confirmed statistically significantly with a primary endpoint and/or secondary endpoints that therapeutic effect is higher in the Compound A group than in the placebo group.

## Claims

1. A therapeutic agent for coronavirus infection comprising 6-fluoro-3-hydroxy-2-pyrazinecarboxamide or a salt thereof as an active ingredient, which is administered to patients with one or more risk factors for disease progression who are at an early stage after onset.

2. The therapeutic agent for coronavirus infection according to claim 1, wherein the patients are patients with novel coronavirus infection.

3. The therapeutic agent for coronavirus infection according to claim 1 or 2, wherein the risk factor for disease progression is selected from the group consisting of advanced age (65 years of age or older), malignant tumor, COPD, heart disease, chronic lung disease, cerebrovascular disorder, chronic kidney disease diagnosed and under treatment, type 2 diabetes mellitus, hypertension or hyperlipidemia, obesity (BMI 30 kg/m² or higher), and smoking habit.

4. The therapeutic agent for coronavirus infection according to any of claims 1 to 3, wherein the onset means that at least one selected from the group consisting of cough, sore throat, headache, nasal obstruction or rhinorrhea, myalgia or arthralgia, fatigue or tiredness, dysgeusia, anosmia, and diarrhea occurs as the first symptom.

5. The therapeutic agent for coronavirus infection according to any of claims 1 to 4, wherein the early stage after onset means within 72 hours from onset of the first symptom.

6. The therapeutic agent for coronavirus infection according to any of claims 1 to 5, wherein the patients are 50 years of age or older.

7. The therapeutic agent for coronavirus infection according to any of claims 1 to 6, wherein 6-fluoro-3-hydroxy-2-pyrazinecarboxamide is administered at 1,000 to 2,400 mg twice a day on Day 1 and at 400 to 1,200 mg twice a day on Day 2 and thereafter.

8. The therapeutic agent for coronavirus infection according to any of claims 1 to 7, wherein 6-fluoro-3-hydroxy-2-pyrazinecarboxamide is administered at 1,800 mg twice a day on Day 1 and at 800 mg twice a day on Day 2 and thereafter.

9. The therapeutic agent for coronavirus infection according to any of claims 1 to 8, which is administered for 10 days.
